# FASCICULE DE BREVET EUROPEEN

(11) **EP 0 536 457 B1**
(45) Date de publication et mention de la délivrance du brevet: **02.01.1997**
(21) Numéro de dépôt: 91440080.9
(22) Date de dépôt: 11.10.1991
(51) Int. Cl.: A61F 2/38

(54) **Prothèse chirurgicale pour l'articulation du genou**
Endoprothese für Kniegelenk
Endoprosthesis for knee joint

(43) Date de publication de la demande: 14.04.1993
(73) Titulaire: SOCIETE CIVILE ESSOR, 74550 Perrignier (FR)
(72) Inventeur: Bosredon, Jean, F-33600 Pessac (FR); Laurent, Patrick, F-47000 Agen (FR); Legroux, Philippe, F-33000 Bordeaux (FR)
(74) Mandataire: Polus, Camille

(56) Documents cités:
- WO-A-85/02535
- US-A- 4 938 769
- US-A- 4 944 757

## Description

La présente invention a pour objet une prothèse chirurgicale pour l'articulation du genou.

Parmi les prothèses connues, certaines comportent un axe d'articulation entre la partie implantée sur le tibia et celle implantée sur le fémur, et d'autres sont à glissement, de façon à simuler l'articulation naturelle. Dans ces dernières les condyles du fémur glissent sur un plateau tibial équipant l'extrémité supérieure du tibia.

Une telle prothèse comprend en général :
- une pièce fémorale comportant deux condyles de part et d'autre d'une gouttière d'axe longitudinal oblique formant la trochlée ;
- un ensemble tibial, dont la plate-forme métallique est surmontée par au moins un plateau de frottement et de glissement amovible, et comportant à sa base au moins une queue d'ancrage ;
- une rotule, pourvue d'une face de glissement sensiblement en forme de calotte sphérique, susceptible de se déplacer le long de la trochlée de la pièce fémorale.

Ce type de prothèse fait notamment l'objet du document FR-A-2 635 679.

La fixation des prothèses est généralement réalisée au moyen de ciment acrylique, mais ce mode de fixation peut être remplacé ou complété par des plots d'ancrages, solidaires de la prothèse, destinés à être insérés dans l'os.

Ainsi le document US-A-4 938 769 décrit une prothèse tibiale dont la plate-forme tibiale est munie de trois perçages permettant de la solidariser, à l'aide de vis, à une tige centrale et à deux plots d'ancrage reliés à ladite tige centrale.

La prothèse de genou décrite dans le document FR-A-2 635 679 comprend une embase tibiale dont la plate-forme métallique présente, sur la face en contact avec l'os, deux plots d'ancrage.

Par ailleurs, en matière de prothèses, on connait aujourd'hui l'utilisation de structures poreuses appliquées sur la face de la prothèse en contact avec l'os afin que la fixation définitive secondaire soit assurée par réhabitation osseuse des pores de ladite structure, la fixation primaire étant réaliséee par exemple au moyen de vis.

Cependant, lorsque l'ablation de la prothèse est nécessaire, la présence de tiges métalliques qui lui sont solidaires rend cette opération très difficile et dommageable pour l'os, l'arrachement des plots de la partie osseuse dans laquelle ils sont insérés occasionnant des lésions.

La présente invention a pour but de remédier à cet inconvénient en proposant une prothèse dont les plots d'ancrage peuvent être facilement sectionnés, afin de permettre l'ablation de la prothèse, les plots restant alors dans la partie osseuse.

La présente invention a ainsi pour objet une prothèse de genou dont la pièce fémorale et l'embase tibiale comportent, solidarisées à leur face en contact avec l'os, une ou plusieurs tiges, ladite prothèse se caractérisant en ce que chaque tige, destinée à accueillir un plot d'ancrage, est filetée et réalisée en polyéthylène permettant de la sectionner facilement en cas d'ablation de ladite prothèse.

D'autre part, la fixation primaire de l'embase tibiale est réalisée à l'aide de vis passées dans quatre orifices pratiqués dans ladite embase et disposés en trapèze, une telle disposition assurant une meilleure fixation dans la partie osseuse.

Les caractéristiques et les avantages de la présente invention ressortiront plus clairement de la description qui suit et qui se rapporte au dessin annexé, lequel en représente un mode de réalisation non limitatif.

Dans le dessin annexé :
- la figure 1 représente une vue de profil, en coupe partielle, d'une embase tibiale selon l'invention.
- la figure 2 représente une vue de dessus de la même embase tibiale.
- la figure 3 représente une vue en perspective d'une pièce fémorale selon l'invention.

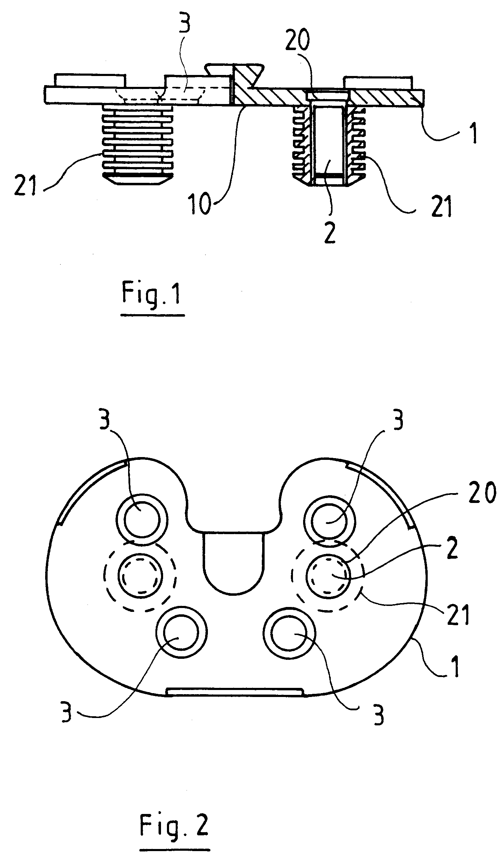

Si l'on se réfère aux figures 1 et 2 on peut voir une embase tibiale 1 comportant deux trous filetés 20 dans lesquels sont vissés deux tiges filetées en polyéthylène 2 sur chacune desquelles est vissé un plot à ailettes en polyéthylène 21. La face 10 de l'embase tibiale 1 en contact avec la partie osseuse est recouverte d'une structure poreuse, et l'embase 1 comporte en outre quatre perforations 3 disposées en trapèze pour le passage de vis.

Ainsi, la fixation primaire de l'embase se fait d'une part à l'aide de vis passées dans les orifices 3 et d'autre part à l'aide des plots en polyéthylène 21 qui sont insérés en force dans l'os, dans des avant-trous légèrement plus petits, les ailettes permettant la progression et la fixation dans ces avant-trous.

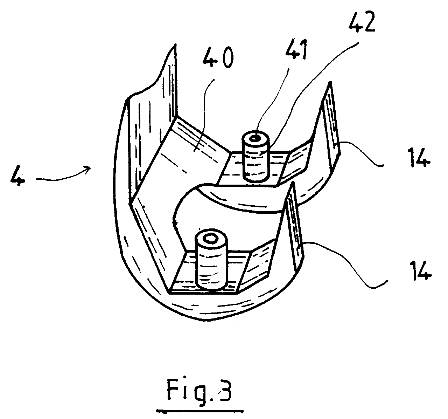

Si l'on se réfère à la figure 3, on peut voir une pièce fémorale 4 comportant, sur sa face 40 destinée à être en contact avec l'os, et au dos des deux condyles 14, deux tiges filetées en polyéthylène 41 sur lesquelles sont vissés deux plots à ailettes en polyéthylène 42, la face 40 étant revêtue d'une structure poreuse, qui peut être de l'hydroxyapatite.

## Revendications

1. Prothèse de genou dont la pièce fémorale (4) et l'embase tibiale (1) comportent, solidarisées à leur face en contact avec l'os, une ou plusieurs tiges (2, 41), caractérisée en ce que chaque tige (2, 41) est filetée et destinée à accueillir un plot d'ancrage (21, 42), et en ce que chaque tige filetée (2, 41) est réalisée en polyéthylène permettant de la sectionner facilement en cas d'ablation de ladite prothèse.

2. Prothèse selon la revendication 1, caractérisée en ce que l'embase tibiale (1) comporte quatre orifices (3) disposés en trapèze pour le passage de vis permettant sa fixation primaire.

## Claims

1. Knee prosthesis the femoral part (4) and tibial base (1) of which comprise, attached to their surfaces in contact with the bone, one or more pins (2, 41), characterised in that each pin (2, 41) is threaded and adapted to accommodate an anchoring stud (21, 42) and in that each threaded pin (2, 41) is made of polyethylene enabling it to be divided into sections with ease in the event of removal of said prosthesis.

2. Prosthesis according to claim 1, characterised in that the tibial base (1) comprises four openings (3) arranged in a trapezium configuration for the insertion of screws for the primary attachment thereof.

## Patentansprüche

1. Knieprothese, deren Schenkelknochenteil (4) und Schienbeinplatte (1) einen oder mehrere Zapfen (2,41) auf ihrer in Berührung mit dem Knochen stehenden Fläche aufweisen, dadurch **gekennzeichnet**, daß jeder Zapfen (2,41) ein Gewinde aufweist und bestimmt ist zur Aufnahme eines Verankerungsteils (21,42), und daß jeder Gewindezapfen (2,41) aus Polyethylen besteht und eine einfache Unterteilung im Falle der Abtrennung ermöglicht.

2. Prothese nach Anspruch 1, dadurch **gekennzeichnet**, daß die Schienbeinplatte (1) vier Öffnungen (3) umfaßt, die im Trapez angeordnet sind und den Durchgang von Schrauben ermöglichen, die die Primärbefestigung gestatten.
